# EUROPEAN PATENT APPLICATION

(11) **EP 2 165 722 A1**
(43) Date of publication of application: **24.03.2010**
(21) Application number: 08164725.7
(22) Date of filing: 19.09.2008
(51) Int. Cl.: A61M 5/145

(54) **Device for feeding a product in a medical or pharmaceutical application**

(71) Applicant: F.HOFFMANN-LA ROCHE AG, 4070 Basel (CH); Roche Diagnostics GmbH, 68305 Mannheim (DE)
(72) Inventor: Fehr, Jean-Noël, 2000 Neuenburg (CH); Troller, Stefan, 4410 Liestal (CH); Von Büren, Thomas, 3008 Bern (CH)
(74) Representative: Schwabe - Sandmair - Marx

(57) **Abstract**

Device for feeding a fluid product in a medical or pharmaceutical application, the device comprising
a) a housing (1) with a compartment (1a) for a product container (2) from which product can be fed by means of a piston (3),
b) a piston rod (15) which is movable axially in a feed direction (F) for feeding the product and in a reverse direction (-F) for retracting the piston rod (15),
c) a drive unit (5) for the piston rod (15),
d) a gear mechanism (6,7,8) coupling the drive unit (5) and the piston rod (15) in a feed state with a feed transmission ratio for moving the piston rod (15) in the feed direction (F),
e) wherein the gear mechanism (6,7,8) can be shifted from the feed state to a reset state in which it couples the drive unit (5) and the piston rod (15) with a reset transmission ratio for moving the piston rod (15) in the reverse direction (-F),
f) the reset transmission ratio being larger than the feed transmission ratio,
g) and an actuator (2) for shifting the gear mechanism (6,7,8).

## Description

The invention is directed to a device for feeding a product in a medical or pharmaceutical application. The device can in particular be a device, preferably an infusion device, for administering a product to a patient, for example to administer insulin or another medical or pharmaceutical substance. Preferred applications of the invention are in therapies in which the patient administer the product to himself.

With devices feeding the product by advancing a piston within a product container the piston rod has to be retracted if an emptied container is to be replaced by a new one. In therapies requiring that the product be administered in small rates the piston is advanced only slowly. Retracting the piston rod is accordingly either tedious or requires a drive motor which can be operated in different modes, one for slowly feeding the product and another one for fast retraction of the piston rod. Such a motor subject to conflicting requirements is linked with high costs or inadequate for the therapy and generally needs additional security features to guarantee the patient security.

It is an object of the invention to provide a device precisely feeding a product in a medical or pharmaceutical application while on the other hand allowing for quick replacement of an emptied product container.

Subject-matter of the invention is a device for feeding a product in a medical or pharmaceutical application, which comprises a housing with a compartment for a product container from which product can be fed by means of a piston, and a piston rod which is movable axially in a feed direction for feeding the product and in a reverse direction for retracting the piston rod. The device furthermore comprises a drive unit for the piston rod and a mechanism which couples the drive unit and the piston rod, in a mechanism feed state, for moving the piston rod in the feed direction.

According to the invention the mechanism can be operated in at least two modes or states, preferably in only two states, namely the feed state and a reset state for retracting the piston rod. The mechanism can be shifted from the feed state to the reset state, and preferably also vice versa. The mechanism couples the drive unit and the piston rod in the feed state with a feed transmission ratio and in the reset state with a reset transmission ratio which is larger than the feed transmission ratio.

Since the mechanism can be operated in different states, the feed state and the faster reset state, the respective product can be fed at a comparatively slow rate and hence precisely matched to the needs of the patient while the piston rod can be retracted faster, as compared with its movement in the feed direction. This enables quick reloading of the device, either by replacing an emptied product container by a prefilled new one, as preferred, or by refilling an emptied container by drawing in fresh product. Therapy becomes more convenient, as is favourable in particular in self administration. A further advantage is that a simple drive unit operating only at one predetermined speed can be used, thereby lowering the cost for the drive unit. The drive unit can be of the reversible type with the same speed in both directions of its drive movement. Alternatively, a drive unit can be used which operates in the same drive direction for feeding the product and for retracting the piston rod. In the latter case the gear mechanism does not only change between the different transmission ratios but does also, upon shifting, reverse the motional direction of a most downstream member of the mechanism, which can directly be the piston rod or a mechanism member which is in direct engagement with the piston rod. In such cases the mechanism can not only be shifted between different transmission ratios but also reversed, preferably at the same time by the same operation.

In applications in which the device is an administering device for administering the product to a human being or, maybe, an animal the feed direction is the direction in which the piston rod is moved towards an outlet of a product container. However, the device may instead be used for drawing a fluid from a source, for example for drawing blood or some other body liquid from a human being or an animal. In such applications the feeding direction is the direction in which the piston rod is moved away from the inlet while retraction is a movement towards the inlet which becomes the outlet for expelling the fluid which has been drawn in before.

The device furthermore comprises an actuator for shifting the mechanism from the feed state to the reset state, and preferably back to the feed state. If the drive unit is of the reversible type, the actuator can be arranged such that it does not only shift the mechanism but also the drive unit.

The actuator can be designed only to shift the mechanism. Such a dedicated actuator is preferably provided accessible at the housing either in or protruding through a hole, groove or some other type of passage formed through the housing. The actuator can for example be a push, turn, pivot or slide button. The actuator can also be designed as to be an automatic switching mechanism and can for example be an electro-magnetic switched release by a pump processor.

In preferred embodiments the actuator is a functional component actuated automatically when replacing the product container by removing a used or inserting a new product container. The word "or" is used in its usual logical meaning, i.e, it is an inclusive or covering the meaning of "either...or" as well as the meaning of "and", as long as the specific context does not rule out one of these meanings without any doubt. The actuator may accordingly be actuated automatically only by inserting a container or only by removing a container or, as preferred, is actuated automatically by insertion and by removal as well. If the compartment is accessible via a closure which has to be opened to remove an emptied container and is closed again after the new container has been inserted, such a closure can constitute the actuator. Preferred are embodiments in which the container as such is the actuator or forms the actuator in combination with such a compartment closure. The compartment closure may be e.g. screwed or plugged in or on the opening of the compartment.

In preferred embodiments the device comprises a shifting structure which is coupled with the actuator at least when manipulating the actuator for shifting the mechanism, or constitutes the actuator. The shifting structure is movable back and forth relative to the housing between a feed position and a reset position. It is preferably axially movable i.e. in and counter to the feed direction. The shifting structure is coupled with the mechanism such that the mechanism is shifted into the feed state when the sifting structure is moved into the feed position and is shifted into the reset state when the shifting structure is moved into the reset position. The shifting structure may constitute the actuator, as mentioned. In such embodiments it can constitute in particular the dedicated actuator mentioned above. However, it is more preferred that the shifting structure is moved into the feed or reset position by means of the actuator which can, in particular, be the product container itself.

The shifting structure can be coupled with the mechanism only electrically or optically, via wire or wireless. In such embodiments the device also comprises a drive means controllable by signals created automatically when the actuator is actuated. In preferred embodiments, however, the shifting structure is mechanically coupled with the mechanism and effects shifting mechanically when being moved from the feed position to the reset position or vice versa.

If the actuator is formed by the product container the shifting structure is preferably accessible via the compartment, even more preferably the shifting structure is located in the compartment. The sifting structure is in such embodiments preferably movable in a direction in which the product container is inserted into the compartment or removed from the compartment. The container is expediently inserted and removed in and counter to the feed direction, i.e. axially. If the container is inserted in a direction transverse to the feed direction, the shifting structure can be movable in and counter to this transverse direction.

The device may comprise a spring exerting a spring force which urges the shifting structure into the feed position or, in alternative embodiments, into the reset position. The spring preferably acts directly on the shifting structure, i.e. is at one of its spring ends in direct contact with the shifting structure although this is not necessarily the case. In principle, the spring may act on the shifting structure indirectly via one or more force transmission element(s). The actuator is preferably acting on the shifting structure against the spring force, either in direct contact with the shifting structure, as preferred, or possibly indirectly via one or more force transmission element(s). In a first embodiment the shifting structure is freely movable back and forth under the influence of the spring force and the counter force of the actuator. In also preferred second embodiments the shifting structure is arrested releasably in a holding position against the spring force and is released by actuating the actuator. The arrested holding position can in particular be the reset position, i.e. the position for retracting the piston rod.

If the product container serves as actuator it preferably presses during insertion or removal against the sifting structure to move the shifting structure into the feed position. The product container may press against the shifting structure via one or more intermediate member(s) or it presses, as preferred, in a direct contact against the shifting structure.

In a first variant of a pressing container action the product container pushes the shifting structure into the feed position when the container is inserted into the compartment. The spring effecting the shifting structure is advantageously disposed such that it urges the shifting structure into the reset position, i.e. counter to the pushing force exerted by the product container. It is preferred that the shifting structure is freely movable back and forth under the effect of the two forces, namely the pushing force of the product container and the restoring spring force.

In a second variant the shifting structure is arrested, relative to the housing, in the reset position by a non-positive lock (frictional fit) or, more preferred, by a positive lock (form fit) when no container is accommodated in the compartment The shifting structure can in particular be engaged with the housing, wherein any structure, like an insert, connected to the housing is regarded as being part of the housing. For arresting the shifting structure in the reset position the shifting structure or the housing may be provided with one or more elastic arresting member(s) engaging in a preferably positive lock to hold the shifting structure in the reset position. The arresting member(s) engage(s) in a snapping action with one or more arresting counter member(s). Both, the shifting structure and the housing, may be provided with one or more elastic arresting member(s) and arresting counter member(s). However, more preferred, only one of the shifting structure and the housing, preferably the shifting structure, is provided with one or more elastic arresting member(s) and the other with one or more rigid arresting counter member(s). The container, upon insertion into the compartment, presses against the elastic arresting member(s), thereby urging the arresting member(s) out of the engagement with the arresting counter member(s) and releasing the shifting structure which now can move into the feed position. In principle, arrest and release of the arrest can also be effected by rigid arresting member(s) and counter member(s) engaging as a cam mechanism.

Preferred embodiments of the second variant comprise the spring which, however, exerts its spring force on the shifting structure, preferably directly or alternatively via one or more force transmission element(s), such that the spring force urges the shifting structure into the feed position once the arrest is released. The same or additional arresting member(s) provided at the shifting structure may engage with the product container such that the product container urges, expediently pulls, the shifting structure back into the reset position when the product container is removed from the compartment. The shifting structure is urged into the reset position against the spring force.

Although preferred, provision of a spring acting to urge the shifting structure into the reset position (first variant) or into the feed position (second variant) is not mandatory. In the second variant, for example, the product container may not only release the arrest but may also push the shifting structure into the feed position during insertion and urge the shifting structure back into the reset position and also into the arrest upon removal. In the first variant an additional actuator may be present, instead of the spring, to urge the shifting structure into the reset position when the product container is removed from the compartment.

The features disclosed with respect to the product container are useful also if the actuator is formed by some other means, for example a closure cap of the compartment. A closure cap can in particular act via the product container, i. e. in combination with the container, e.g. in the variants outlined above by pressing the product container against the shifting structure when closing the compartment. Alternatively, a closure cap can act onto the shifting structure while circumventing the product container. However, such a mechanism would require additional parts and is therefore less preferred although totally within the scope of the invention.

The shiftable mechanism comprises a plurality of mechanism members, at least three mechanism members two of which are connected, alternating upon shifting, to the at least third one, preferably by an alternating direct engagement. More preferred, the mechanism comprises at least four members, namely a first drive member, a second drive member, a first driven member and a second driven member. The first driven member is allocated to the first drive member and the second driven member is allocated to the second drive member such that by shifting the mechanism from the reset state to the feed state or vice versa either the first members or the second members are in driving connection, preferably in mutual engagement, whereas the other two allocated mechanism members are disconnected. The respective pair of engaged members transmits the driving motion of the drive unit to the piston rod, whereas the respective other pair of mechanism members is disconnected. One of the two pairs of allocated members transmits the driving motion at the feed transmission rate and the other pair at the reset transmission rate.

The mechanism may be of any type, for example a cam mechanism. Preferably, the mechanism is a gear mechanism or comprises a gear mechanism and the mechanism members mentioned above are gear wheels. A gear mechanism can advantageously be combined with a drive unit of the rotary type. The wheels allocated in pairs are preferably mating one with the other of the respective pair depending on the state of the mechanism. If the mechanism comprises at least three gear wheels two of these gear wheels can be provided on a drive shaft, for example the rotor of a rotary type drive unit or a drive shaft further down in the transmission chain, and the third gear wheel on a driven shaft, for example the piston rod or a driven shaft further up in the chain but down from the drive shaft. Alternatively, only one of these gear wheels can be provided on the drive shaft and the two others on the driven shaft. When shifting the mechanism between the feed and the reset state the two gear wheels on the common shaft, either the drive shaft or the driven shaft, are alternatingly connected to the respective third gear wheel, preferably engaged with the third wheel, i.e. without any intermediate transmission member.

The driven shaft is preferably engaged with the piston rod. The engagement can be a thread engagement wherein the piston rod is preferably provided with an external thread and the driven shaft with an internal thread. Alternatively, the piston rod and the driven shaft can be engaged such that they cannot rotate one relative to the other about the longitudinal axis of the piston rod but can move one relative to the other in and counter to the feed direction which coincides with the longitudinal axis of the piston rod. In both cases, the thread and the guided linear engagement, the piston rod is in a further engagement which effects axial movement of the piston rod when the driven shaft is rotated. This further engagement is preferably with the housing. In the further engagement the piston rod can in particular be guided axially secured against rotation about its longitudinal axis. This can be the case in particular if the engagement of the piston rod and the driven shat is a thread engagement. The further engagement can alternatively be a thread engagement, in particular if the piston rod is axially guided by the driven shaft and secured against rotation relative to the driven shaft but also in cases in which the engagement with the driven shaft is a thread engagement. In the latter case the thread lead of the two engagements are different.

In embodiments in which the shiftable mechanism comprises a first and a second drive gear wheel these wheels are preferably provided on a common drive shaft securely mounted against rotation about the shaft axis or formed in one piece with the drive shaft. In embodiments in which the gear mechanism comprises a first and a second driven gear wheel these gear wheels are provided on a common driven shaft securely mounted against rotation about the shaft axis or formed in one piece with the driven shaft.

In embodiments in which the driven unit is a rotary motor the rotor of the motor can form the drive shaft. If the driven shaft is engaging the piston rod, the gear mechanism is composed of only two stages, the drive and the driven stage. Even the piston rod as such may constitute the driven shaft. In preferred embodiments, however, the driven shaft is engaged with and not constituted by the piston rod and the gear mechanism comprises at least one intermediate stage between the rotor of the drive unit and the driven shaft, preferably only one intermediate stage, formed by the drive shaft.

In principal, the mechanism can be an infinitely variable mechanical transmission. More preferred is a mechanism which can be shifted between certain states including the feed state and the reset state, and in a most preferred basic embodiment the mechanism can be sifted back and forth only between these two states. In those embodiments in which the mechanism is a gear mechanism with at least three or more preferred at least four gear wheels, shifting from one state into the other is accomplished by moving the driven shaft or the drive shaft axially, one relative to the other, thereby drivingly connecting those gear wheels, drive gear wheel and driven gear wheel, which are allocated one to the other in the respective state of the mechanism. With only three gear wheels it might be necessary to move one of the shafts, or both, not only axially but also transversely relative to the respective other shaft.

It should be mentioned with respect to the gear wheels that it is preferred that the gear wheels connected in the feed state are toothed gear wheels. The gear wheels connected in the reset state may be formed as friction wheels but are preferably toothed gear wheels, too.

The drive unit can be of any type, at least in principal It is preferably of the rotary type and can in particular be a rotary motor. The driving motion of the drive unit can be generated e.g. pneumatically with a pressure fluid reservoir or mechanically e.g. by means of a pretensioned mechanical spring or, more preferred, electrically. Most preferred is an electric rotary motor, for example, a stepper motor.

The drive unit can be of the reversible type but can be in principal also be a one-directional, i.e. non-reversible drive. In embodiments in which a one-directional drive unit is used the mechanism preferably comprises a first number of gear stages to transmit the rotary drive motion in the feed state and a second number of gear stages for transmitting the drive motion in the feed state, the first number and the second number differing by an uneven integer, preferably by one, in order to reverse the direction of the rotational motion transmitted to the piston rod.

In embodiments in which the drive unit is reversible the actuator or the shifting structure can be arranged such that upon actuation it switches the drive unit from one direction of its drive motion to the other. In principal, an additional switch can be provided for changing the motional direction of the drive unit. Shifting the mechanism and switching a reversible drive unit by means of the same actuator is, however, preferred, not the least for safety reasons. In this respect it is preferred that the product container or some other component which must necessarily be manipulated for replacing the product container serves as the actuator and also for reversing the direction of the drive motion. For example, the drive unit may be responsive to a movement of the product container, i.e. a movement in the feed direction or a movement in the reset direction, or it maybe actuated by detection of a contact of an arresting means. Alternatively, the drive direction may be manipulated by a user action at a user interface, for example, it may be possible for the user to select a specific menu point when changing the product container to actuate the drive unit.

Preferred features are also disclosed in the sub-claims and by combinations of sub-claims.

The invention will now be explained by way of example embodiments shown in figures. The features disclosed by the example embodiments, each feature individually and in any combination of features, develop the subjects of the claims and also the embodiments discussed above. There is shown:
- Figure 1: a device for administering a product according to a first example embodiment, in a perspective view;
- Figure 2: a longitudinal section of the device of the first example embodiment;
- Figure 3: a feeding system of the device of the first example embodiment in a feed state;
- Figure 4: the feeding system of the first example embodiment in a reset state;
- Figure 5: a device for administering a fluid product according to a second example embodiment;
- Figure 6: a first longitudinal section of the device of the second example embodiment, a feeding system of the device being in a reset state;
- Figure 7: the device of the second example embodiment in a second longitudinal section which is perpendicular to the first longitudinal section;
- Figure 8: a detail of figure 7;
- Figure 9: the device of the second example embodiment in the first longitudinal section, the feeding system being in the feed state;
- Figure 10: the device of the second example embodiment in the second longitudinal section, the feeding system being in the feed state;
- Figure 11: a detail of figure 10;
- Figures 12-15: a sequence of shifting the feeding system of the second example embodiment into the reset state; and
- Figur 16: a modified gear mechanism.

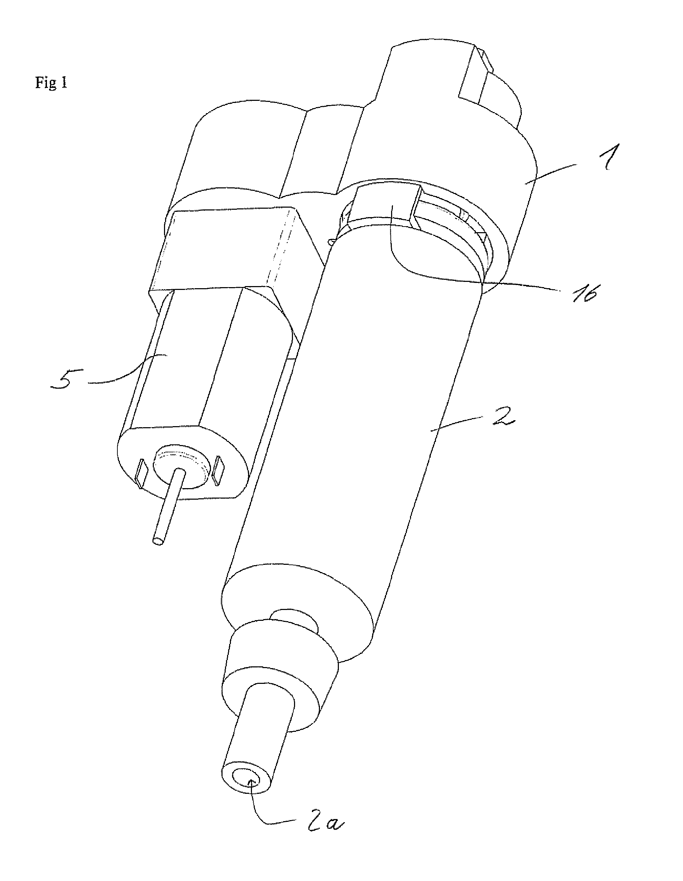

Figure 1 shows a device for administering a fluid product e.g. insulin. An outer shell of the housing and electronic components of the device are omitted. Of the housing only a base 1 is shown supporting a product container 2 and a feeding system for feeding the product through an outlet 2a of the container 2. Of the feeding system a drive unit 5, preferably an electric motor e.g. stepper motor, and part of' a shifting structure 16 are shown.

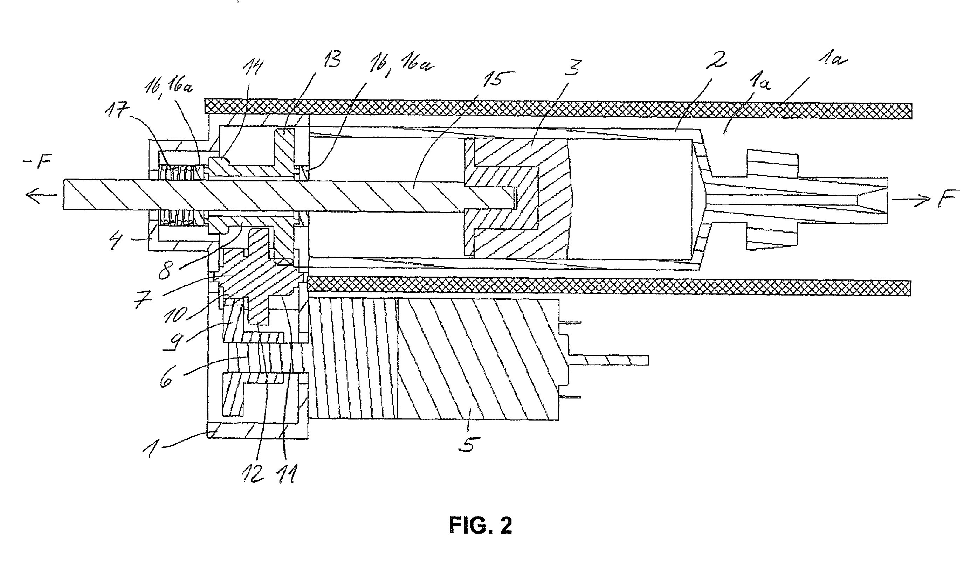

Figure 2 shows the product container 2 and the feeding system in a longitudinal section. The feeding system comprises the drive unit 5, a piston 3 accommodated in the container 2 movable axially in a feed direction F towards the container outlet 2a to expel the product, a piston rod 15 and a mechanism for transmitting a drive movement of the drive unit 5 to the piston rod 15 for moving the piston rod 15 and accordingly the piston 3 in the feed direction F. The drive unit 5 is a rotary motor generating a rotary drive movement. The mechanism is a gear mechanism comprising a plurality of shafts 6, 7 and 8 forming gear stages for transmitting the drive movement at a certain transmission ratio to the most downward driven shaft 8 which is drivingly coupled directly with the piston rod 15. The driven shaft 8 is coupled with the piston rod 15 by thread engagement. The piston rod 15 has an external thread over almost its entire length and the shaft 8 is internally threaded, shaft 8 and piston rod 15 thereby forming a nut and screw drive 8, 15 for the piston rod 15. The base 1 guides the piston rod 15 axially movable but secured against rotation about the thread axis of the nut and screw drive 8, 15.

The container 2 is replaceably accommodated in a compartment 1a of the housing of which a shell portion 1b is shown. The base 1 is located at the bottom of the compartment 1a. For replacing an emptied container 2 a closure cap is removed from an opening of the compartment 1a, the emptied container 2 is pulled out axially, in the feed direction F, through the opening, a new container 2a is inserted and the opening is closed again by the closure cap. The container 2 is arrested in the compartment 1a axially between the base 1 and the closure cap. The container 2 is prefilled with the product, the piston 3 being accommodated in the container 2 for closing it to the rear, By inserting the container 2 the piston rod 15 is connected to the piston 3 for advancing the piston 3 during administration.

The feeding system is reversible to advance the piston 3 in the feed direction F for administering the product and to retract the piston rod 15 in the reverse direction -F for replacing an emptied container 2. The drive unit 5 is reversible for advancing and retracting the piston rod 5. The drive unit 5 is designed to operate at a maximum speed adjusted to the needs of the administration. This maximum speed is relatively low and is the same in both directions of the rotary drive movement. In the example embodiment the maximum speed of the drive unit 5 is constant and is identical in both directions, as is the case in most of such devices e.g, an infusion pump for self-administration in diabetes therapy. A drawback of a low feed speed is an accordingly low speed in the reverse direction -F for retracting the piston rod 15. Resetting conventional devices, like insulin pumps, has therefore been a tedious process.

To advance the piston 3 as slowly as required by the specific therapy in the feed direction F but to retract it in the reverse direction -F for resetting the device, the mechanism 6, 7, 8 can be shifted back and forth between a feed state and a reset state. The mechanism 6, 7, 8 transmits the speed of the drive unit 5 at a reset transmission ratio in the reset state, the reset transmission ratio being larger than a feed transmission ratio at which the mechanism 6, 7, 8 transmits the speed of the drive unit 5 in the feed state. The transmission ratio in the reset state is preferably at least twice the transmission ratio in the feed state. For example, the reset transmission ratio can approximately be 1:150 and the feed transmission ratio approximately 1:600.

In figure 2 the feeding system is illustrated with the mechanism 6, 7, 8 in its feed state. In the feed state the drive movement of the drive unit 5 is transmitted from the output shaft 6 via a primary gear wheel 9 provided on the motor shaft 6 to a secondary gear wheel 10 provided on the drive shaft 7. A first drive gear wheel 11 also provided on the drive shaft 7 is drivingly connected with a first driven gear wheel 13 provided on the driven shaft 8. The first wheels 11 and 13 are directly engaged, i.e. they mesh one with the other in the feed state of the mechanism 6, 7, 8. Since the piston rod 15 is linearly guided but secured against rotation relative to the base 1 and is in thread engagement with the driven shaft 8, the piston rod 15 advances in the feed direction F upon rotation of the driven shaft 8, thereby expelling metered amounts of the product through the outlet 2a.

The drive shaft 7 and the driven shaft 8 are provided with additional gear wheels, the drive shaft 7 with a second drive gear wheel 12 and the driven shaft 8 with a second driven gear wheel 14. The second wheels 12 and 14 are disposed axially offset from the respective first wheel 11 or 13 on the respective shaft 7 or 8. By shifting the shafts 7 and 8 axially, one relative to the other, either the first wheels 11 and 13 or the second wheels 12 and 14 can drivingly be connected while the other pair of wheels is disconnected.

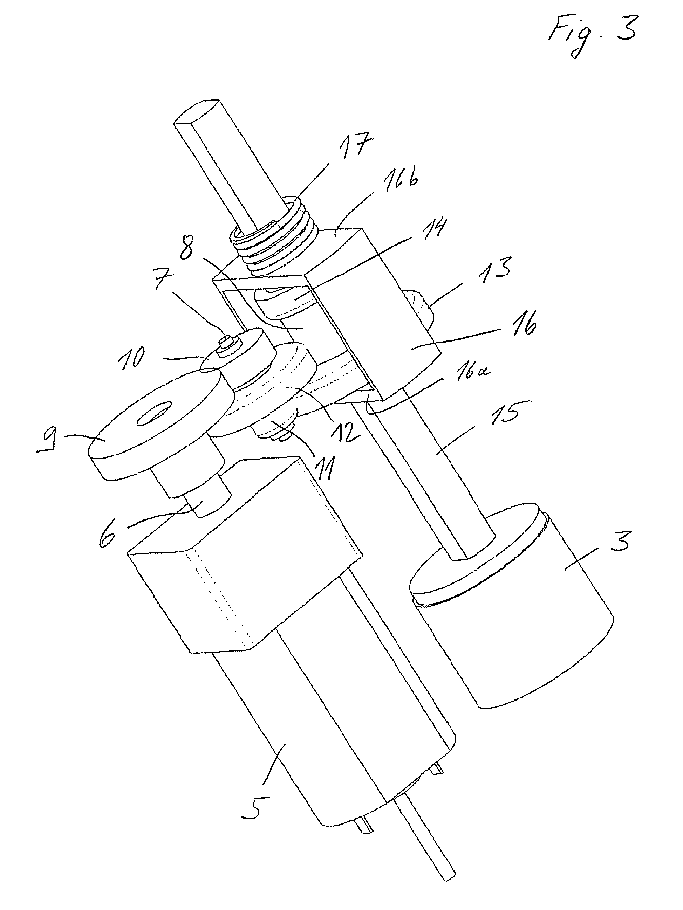

Figure 3 shows the feeding system in a perspective view. The base 1 has been omitted. The mechanism 6, 7, 8 is in its feed state, as in figure 2.

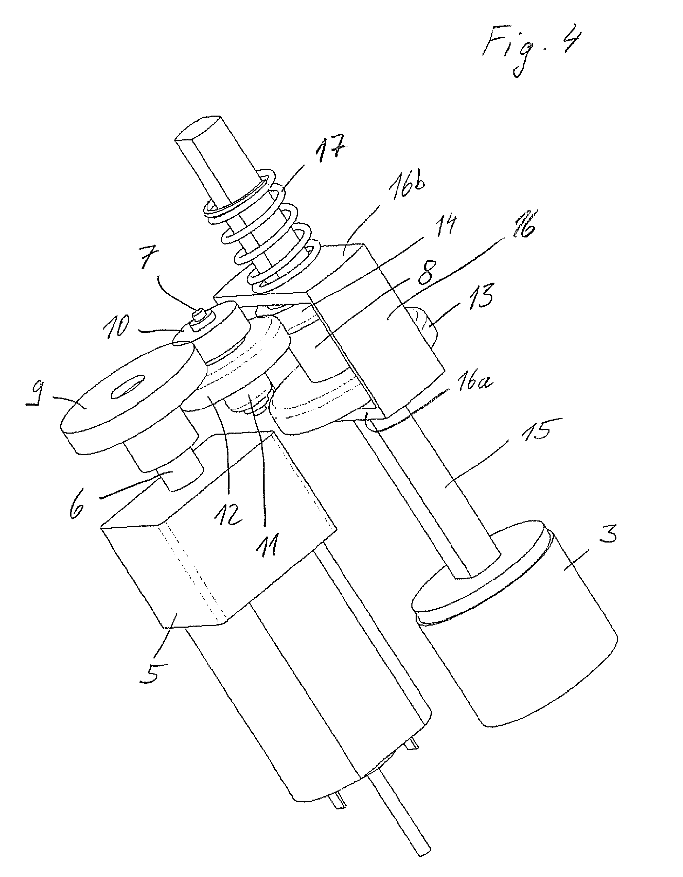

Figure 4 shows the feeding system in the same perspective view as figure 3. Again, the base 1 has been omitted. The mechanism 6, 7, 8 has been shifted to its reset state by shifting the drive shaft 7 and the driven shaft 8 one relative to the other axially. By the relative shifting movement the first wheels 11 and 13 have been disconnected and the second wheels 12 and 14 drivingly connected by bringing the first wheels 11 and 13 out and the second wheels 12 and 14 into an axial overlap in which they mate.

The axial shifting movement is a relative movement of the shafts 7 and 8. In principal both of the shafts 7 and 8 could be shifted relative to the base 1 or, alternatively, the drive shaft 7 could be mounted shiftably. In the example embodiment, however, only the driven shaft 8 is shiftable relative to the base 1 and the shaft 7, in particular the drive wheels 11 and 12, are axially fixed relative to the base 1. The primary gear wheel 9 and the secondary gear wheel 10 are permanently drivingly connected therefore. If the drive shaft 7 would have to be shifted, this shaft might be axially movable relative to gear wheel 10 while being secured against relative rotation, or gear wheel 9 or 10 might have a greater axial length to maintain engagement. In yet a further alternative an additional gear wheel coupled to the shaft 6 could be engaged in order to further increase the reset transmission ratio.

The driven shaft 8 is supported shiftably axially by means of a shifting structure 16 such that it can freely rotate relative to the shifting structure 16 but is moving axially together with the shifting structure 16. The shifting structure 16 comprises a front member 16a facing the rear side of the container 2 and forming a bottom of the compartment 1a on which the container 2 is seated with a rear container face. The shifting structure 16 furthermore comprises a rear member 16b axially offset from the front member 16a. The driven shaft 8 is disposed between the front and the rear members 16a und 16b of the shifting structure 16.

The feeding system furthermore comprises a spring 17 which exerts a spring force on the shifting structure 16 in the feed direction F. The spring 17 is supported at one end by the rear member 16b of the shifting structure 16 and at its other end by a rear shoulder 4 of the base 1 and is thereby tensioned axially between the base 1 and the shifting structure 16. The spring 17 is with respect to form a helical spring and by function a compression spring, but may be substituted by some other type of mechanical spring or alternatively by a gas compression spring. The shifting structure 16 comprises axial webs connecting the front member and the rear member thereby forming a closed frame around the driven shaft 8. However, the shifting structure 16 can in principal be composed only of the front member 16a and the rear member 16b the primary purpose of which are to decouple the container 2 and the spring 17 from the rotary movement of the driven shaft 8. In principal, the driven shaft 8 might constitute also the shifting structure, in particular if friction between the rotating shaft 8 on the one and the container 2 and the spring 17 on the other hand could be kept at a tolerable level.

The mechanism 6, 7, 8 is shifted automatically by a manipulation regularly necessary to replace the product container 2. The product container 2 represents, when being inserted into and also when being removed from the compartment 1a, an actuator actuation of which causes the mechanism 6, 7, 8 to shift from the feed state to the reset state and from the reset state to the feed state. Insertion of the product container 2 into the compartment 1a is an actuation by which the mechanism 6, 7, 8 is shifted from the reset state into the feed state shown in figures 2 and 3 Removal of the product container 2 is an actuation by which the mechanism 6, 7, 8 is shifted from the feed state into the reset state shown in figure 4. For removal the container 2 is moved in the feed direction F away from the base 1 through the opening of the compartment 1a. The tensioned spring 17 forces the shifting structure 16 and therewith the driven shaft 8 to move axially in the feed direction. The first wheels 11 and 13 are moved out of engagement, i.e. the feed gear train 9, 10, 11 and 13 is interrupted, and the second wheels 12 and 14 are engaged to be drivingly connected, i.e. the reset gear train 9, 10, 12 and 14 is closed. The shifting structure 16 can be moved forth and back about a certain axial distance which is predetermined by abutment of axial stops of the base 1 or some other part of the housing. During removal of the container 2 the piston rod 15 is disconnected from the piston 3, either automatically if the piston rod 15 is only plugged into the piston 3 or by turning the container 2 if the piston rod 15 is in thread engagement with the piston 3.

The drive unit 5 is reversed by actuating a switch, preferably an electrical switch. The device can be provided with a switch at the housing, which can be actuated by the user. Alternatively, the shifting structure 16 can function as such a switch to reverse the drive unit 5 automatically when shifting the mechanism 6, 7, 8. After the piston rod 15 has been disconnected from the piston 3, the mechanism 6, 7, 8 been shifted into the reset state and the drive unit 5 been reversed the piston rod 15 is retracted relative to the base 1 into a retracted axial position. Such a switch can be actuated either by the user or by a microcontroller of the pump as the result of the detection of the piston rod 15 disconnecting the piston 3, alternatively as the detection of the removal of the cartridge or alternatively as the result of a process initiated by the user.

A new container 2 is inserted into the compartment 1a with the piston rod 15 in its retracted position. During insertion the piston rod 15 is connected with the piston 3 of the new container 2 and the container 2 presses with its rear face against the shifting structure 16 which constitutes a axially movable bottom of the compartment 1a. The container 2 pushes the shifting structure 16 against the force of the spring 17 into the feed position, shown in figures 2 and 3, in which the reset gear train 9, 10, 12, 14 is interrupted and the feed gear train 9, 10, 11, 13 is closed. In embodiments in which the shifting structure 16 fulfils the double function of shifting the mechanism 6, 7, 8 and reversing the drive unit 5 the device is ready for administering the product. If the device comprises an additional switch assigned to reverse the drive unit 5, this switch is actuated to bring the feeding system into the feed state.

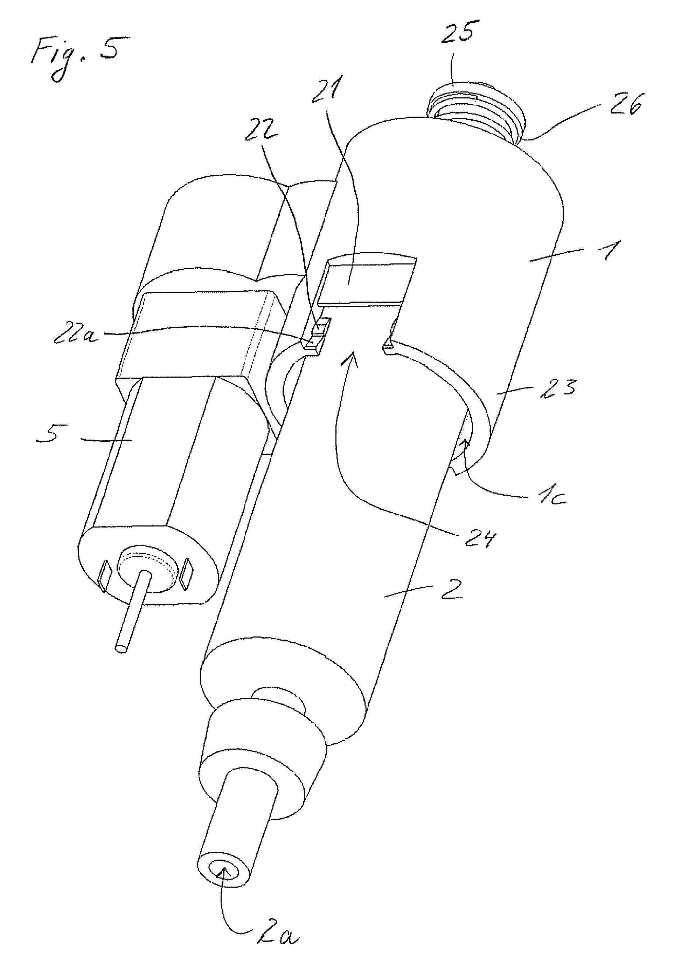

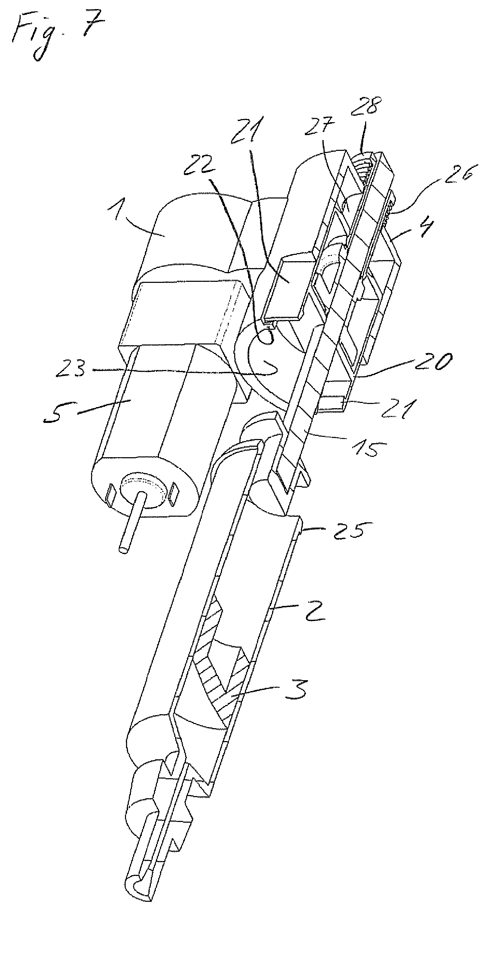

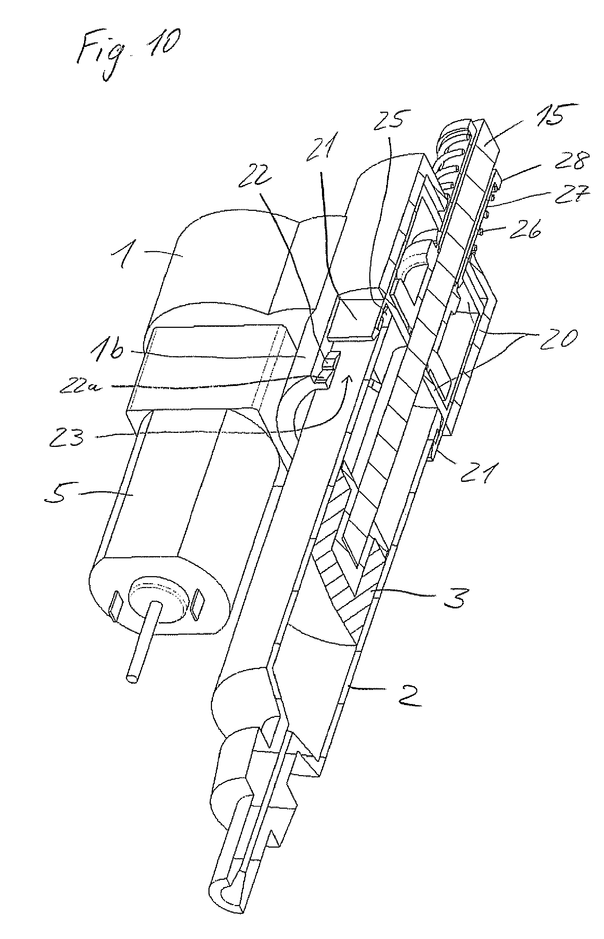

Figures 5 to 11 illustrate a second example embodiment of a device for administering the product. Again, only the feeding system of the second example embodiment and the product container 2 are shown. The feeding system is similar to that of the first example embodiment, in particular the drive unit 5, the gear mechanism 6, 7, 8 with its plurality of gear wheels 9-14 and the piston rod 15 are identical to those members of the first example embodiment. The second example embodiment is different only with respect to the direction of the spring force which acts on the mechanism 6, 7, 8 in the second embodiment to shift it into the feed state. The spring is given the reference numeral 26. The spring 26 is a helical compression spring, as in the first example embodiment, only its arrangement is different in order to create a spring force acting on a shifting structure 20 into the reverse direction -F, i.e. counter the feed direction F. Those parts of the device which are identical or can be regarded as being identical to the respective parts of the first example embodiment have been given the same reference numerals. With respect to these parts reference is made to the explanations to the first example embodiment. Only the differences will be explained.

The shifting structure 20 is arrested in the reset position, against the spring force. The arrest of the shifting structure 20 is effected by a positive lock of the shifting structure 20 and the base 1. The lock is releasable by pressing the container 2 upon insertion into the compartment 1a, against the shifting structure 20, namely against arresting members of the shifting structure 20 which are positively engaged with arresting counter members of the base 1.

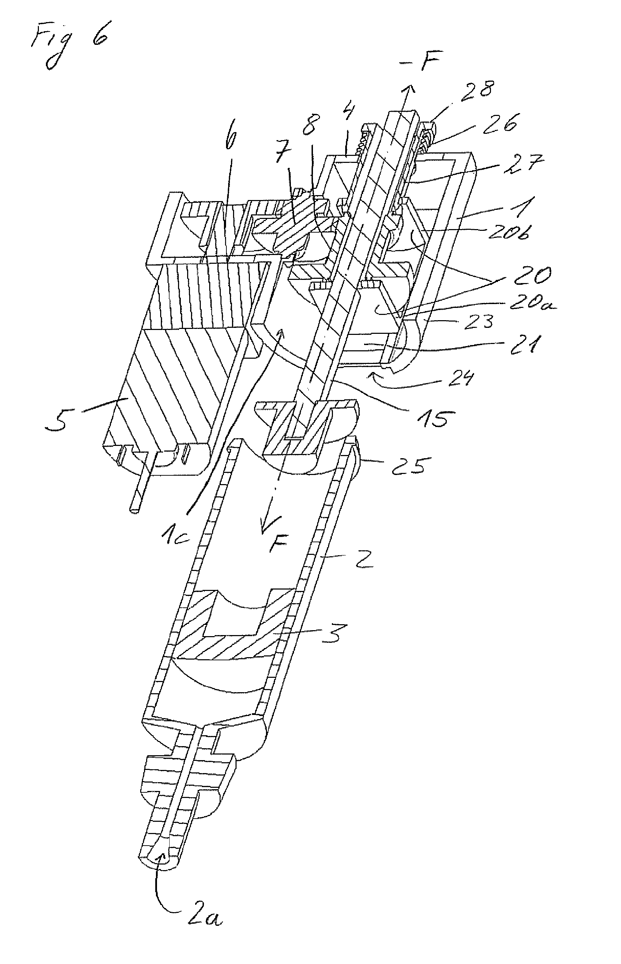

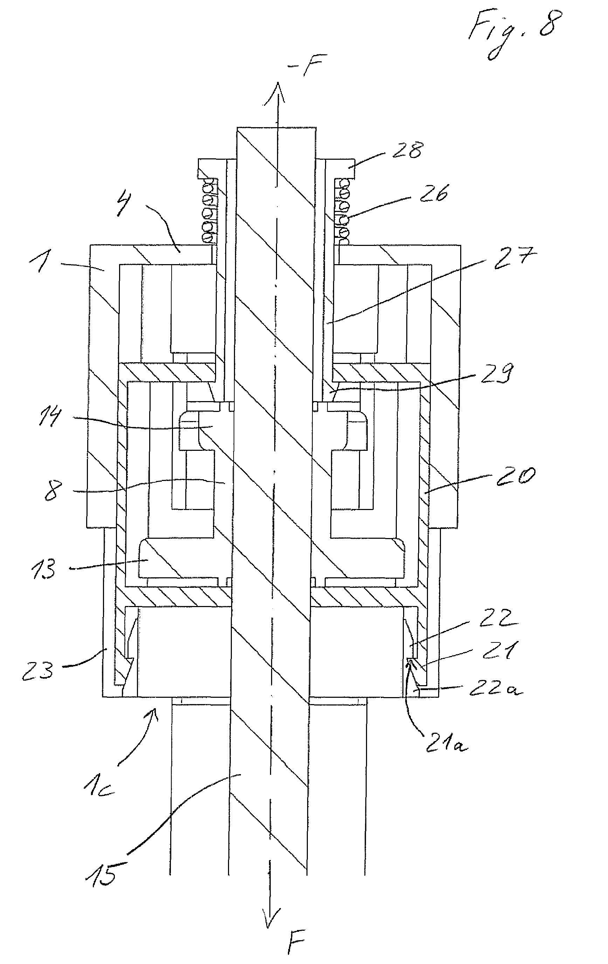

In figures 6 to 8 the feeding system is shown in the reset state. In the reset position the shifting structure 20 is arrested by the interaction of arresting members 21 provided at the shifting structure 20 and arresting counter members 22 provided at the base 1. The interaction can best be seen in figure 8. The arresting members 21 comprise hooks with abutment surfaces 21a by means of which the arresting members 21 grip behind abutment counter surfaces of the arresting counter members 22. In total, the shifting structure 20 is provided with two arresting members 21 spaced apart angularly about 180° around the central longitudinal axis of the piston rod 15. The base 1 is accordingly provided with arresting counter members 22. The arresting counter members 22 are formed in a frontal sleeve section 23 of the base 1 protruding into the compartment 1c. Furthermore, the arresting counter members 22 are disposed in recesses 24 formed in the sleeve section 23 and extending axially from a frontal rim of the sleeve section 23 into the reverse direction -F. The recesses 24 are broader than the arresting counter members 22 in the circumferential direction of the sleeve section 23 thereby leaving space circumferentially to the side of the arresting counter members 22. The arresting members 21 are circumferentially broader than the arresting counter members 22. They protrude further radially inwards in the region of the recesses 24 aside the arresting counter members 22 so that they protrude radially before the inner circumferential surface of the sleeve section 23. The container 2 is provided at its rear face with an outer flange 25 protruding radially outwards over the entire circumference of the container 2. When inserting the container 2 the flange 25 is pressed against the arresting members 21 in those circumferential regions in which the arresting members 21 protrude radially inward before the sleeve section 23 of the base 1. The arresting members 21 and the container flange 25 are formed such that the flange 25 can smoothly slide beyond the inward radial protrusions of the arresting members 21 thereby pressing the arresting members 21 radially outward out of the engagement with the arresting counter members 22. The spring 26 now urges the released shifting structure 20 in the reverse direction -F. As soon as the container 2 has been inserted far enough that its flange 25 is axially beyond the arresting counter members 22, the arresting members 21 snap behind the flange 25.

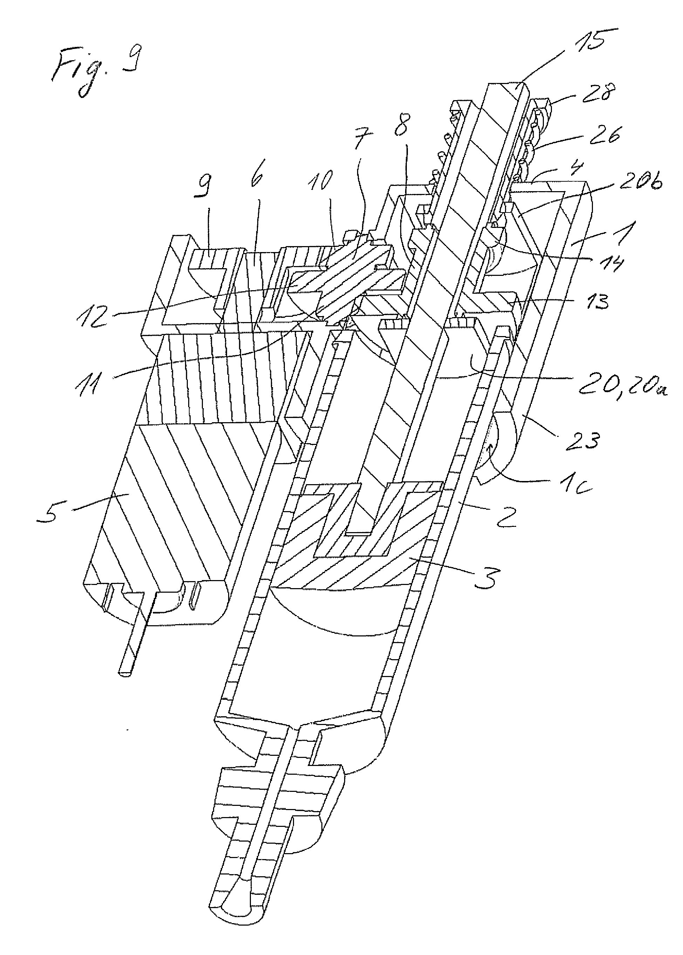

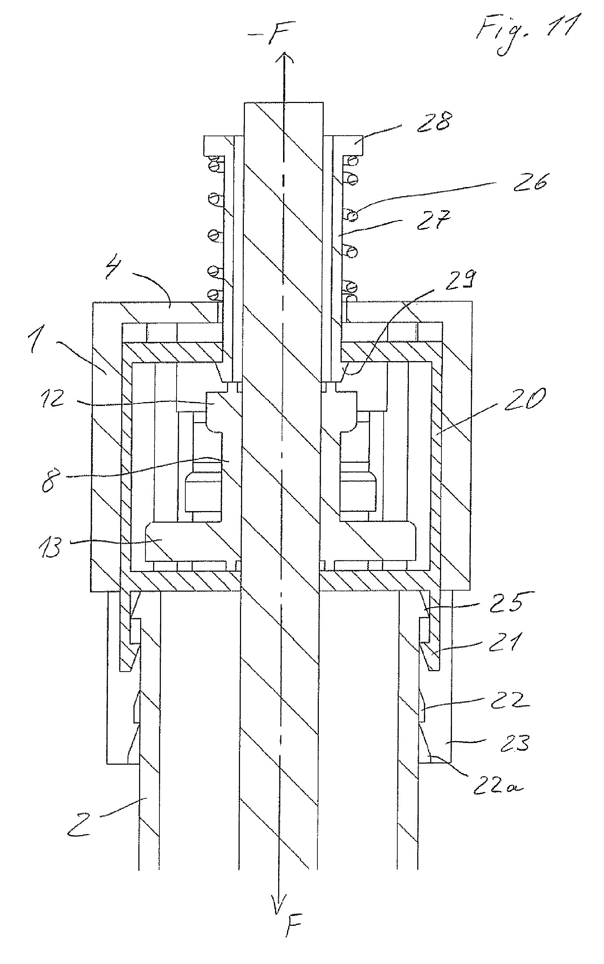

Figures 9 to 11 show the feeding system in the feed state. The container 2 is fully inserted into the compartment 1a. Co-operation of the shifting structure 20, the base 1 and the container 2 for arresting and releasing the shifting structure 20 can best be seen when comparing figures 8 and 11. Figure 5 on the other hand illustrates the arrangement of the arresting counter members 22 and liberation means 22a in the respective recesses 24 of the base 1.

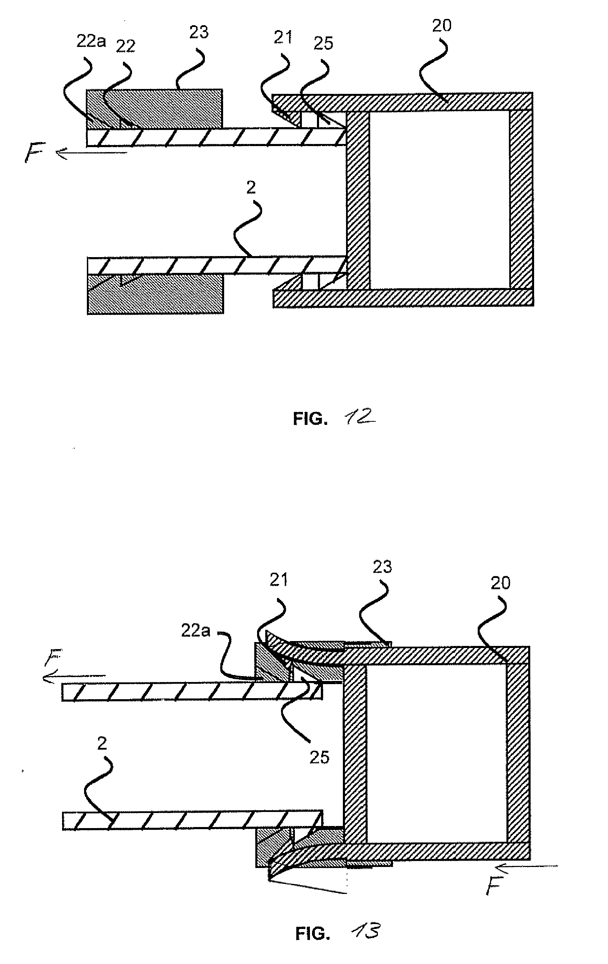

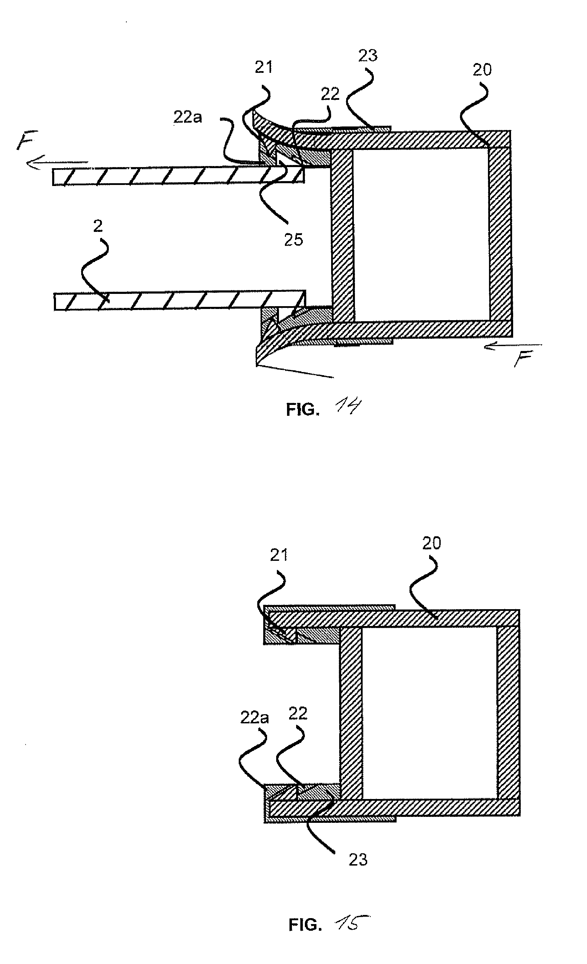

When the container 2, as shown in figure 12, is moved into the feed direction F to remove it from the compartment 1c the rear flange 25 abuts against the arresting members 21 axially pulling the shifting structure 20 into the feed direction F against the force of the spring 26 and relative to the sleeve section 23. As soon as the arresting members 21 reach the arresting counter members 22 these force the arresting members 21 to bend elastically outwards, as shown in figure 13, allowing the arresting members to pass the counter member 22. Figure 13 shows the arrangement at the moment the arresting members 21 pass the arresting counter members 22. The arresting members 21 stay in engagement with the flange 25 while passing the counter members 22 in the feed direction F. When the arresting members 21 reach the liberation means 22a, as shown in figure 14, these force the arresting members 21 to bend elastically outwards further, since the liberation means 22a protrude radially a certain distance beyond the arresting counter members 22, thereby disengaging the arresting members 21 from the container 2, i.e. the container flange 25. This is shown in figure 14. When the flange 25 has passed the arresting members 21 these snap back behind the arresting counter members 22 thereby arresting the shifting structure 20 again in the reset position as shown in figure 15.

In the second example embodiment the spring 26 does not act directly on the shifting structure 20 but only via an intermediate structure 27. The intermediate structure 27 protrudes through the rear wall 4 of the base 1. It comprises a radially protruding rear flange 28 and a radially protruding front flange 29. The spring 26 is tensioned between the rear wall 4 of the base 1 and the rear flange 28. The intermediate structure 27 protrudes through the rear member 20b of the shifting structure 20 and grips behind this rear member 20b. In this manner the spring force acts into the reverse direction -F on the intermediate member 27 which forces, via the front flange 29, the shifting structure 20 into the reverse direction -F. The shifting structure 20, on the other hand, forces the intermediate member 27, via the engagement with the front flange 29, into the feed direction F, against the spring force, when the shifting structure 20 is pulled into the feed direction F by means of the container 2.

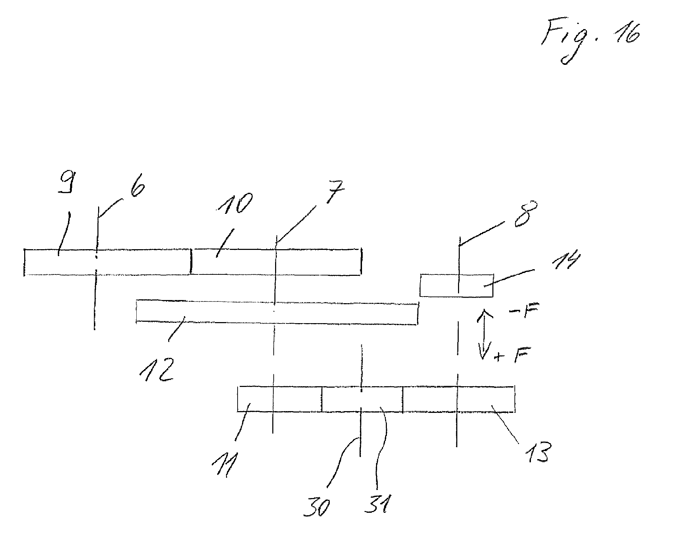

Figure 16 shows schematically a modified gear mechanism which when shifted does not only change the transmission ratio but also the direction of the rotary movement of the driven shaft 8 without reversing the drive unit 5. The modified mechanism differs from the mechanism 6, 7, 8 of the first and the second example embodiment in that it comprises an additional shaft .30 which is provided with an additional gear wheel 31 that drivingly connects the drive shaft 7 and the driven shaft 8 in the feed state. This results in a feed gear train 9, 10, 11, 31 and 13 which is "longer" than the reset gear train 9, 10, 12 and 14, namely by the additional wheel 31. In a further modification, not illustrated, an additional wheel may be inserted in the reset gear train instead of inserting the additional gear wheel 31 in the feed gear train.

The modified mechanism 6, 7, 30, 8 may substitute the gear mechanism 6, 7, 8 in all embodiments of the invention, in particular in the first and second example embodiment shown in the figures and outlined above with respect to the figures. In a device comprising the modified gear mechanism 6, 7, 30, 8 a reversible drive unit 5 is not needed and may, but must not, be substituted by a one directional drive unit.

### Reference numerals:

- 1: housing, base
- 1a: compartment
- 1b: housing, shell portion
- 1c: compartment
- 2: container, actuating member
- 2a: outlet
- 3: piston
- 4: support, rear wall
- 5: drive unit
- 6: motor shaft
- 7: drive shat
- 8: driven shaft
- 9: primary wheel
- 10: secondary wheel
- 11: first drive wheel
- 12: second drive wheel
- 13: first driven wheel
- 14: second driven wheel
- 15: piston rod
- 16: shifting structure
- 16a: front member
- 16b: rear member
- 17: spring 18 -
- 19: -
- 20: shifting structure
- 20a: front member
- 20b: rear member
- 21: arresting member, snapper
- 21a: abutment surface
- 22: arresting counter member
- 22a: liberation means
- 23: sleeve section
- 24: recess
- 25: flange
- 26: spring
- 27: intermediate structure
- 28: rear flange
- 29: front flange
- 30: additional drive shaft
- 3: additional drive wheel
- F: feed direction
- -F: reset direction

## Claims

1. Device for feeding a fluid product in a medical or pharmaceutical application, the device comprising
a) a housing (1) with a compartment (1a) for a product container (2) from which product can be fed by means of a piston (3),
b) a piston rod (15) which is movable axially in a feed direction (F) for feeding the product and in a reverse direction (-F) for retracting the piston rod (15),
c) a drive unit (5) for the piston rod (15),
d) a mechanism (6, 7, 8) coupling the drive unit (5) and the piston rod (15) in a feed state with a feed transmission ratio for moving the piston rod (15) in the feed direction (F),
e) wherein the mechanism (6, 7, 8) can be shifted from the feed state to a reset state in which it couples the drive unit (5) and the piston rod (15) with a reset transmission ratio for moving the piston rod (15) in the reverse direction (-F),
f) the reset transmission ratio being larger than the feed transmission ratio,
g) and an actuator (2) for shifting the gear mechanism (6, 7, 8).

2. Device according to the preceding claim,
further comprising a shifting structure (16) which is coupled with the actuator (2) at least when actuating the actuator (2) or constitutes the actuator (2),
the shifting structure (16) being movable relative to the housing (1), preferably axially, between a feed position and a reset position
and being coupled with the mechanism (6, 7, 8) such that the mechanism (6, 7, 8) is shifted into the feed state when the shifting structure (16) is moved into the feed position and is shifted into the reset state when the shifting structure (16) is moved into the reset position.

3. Device according to any of the preceding claims, further comprising a spring (17) exerting a spring force which urges the mechanism (6, 7, 8) into the feed state or the reset state, preferably urging the shifting structure (16) of claim 2 into the feed position or into the reset position.

4. Device according to the preceding claim in combination with claim 2 and at least one of the following features:
- the actuator (2) is acting on the shifting structure (16) against the spring force;
- the actuator (2) is acting on the shifting structure (16) against the spring force and the shifting structure (16) can be moved back and forth freely between the feed position and the reset position under the action of the actuator (2) and the spring (17);
- the spring force urges the shifting structure (16) into the reset position and the actuator (2) urges, preferably pushes, the shifting structure (16) into the feed position;
- the shifting structure (16) is arrested against the spring force by arresting the actuator (2);
- the spring force urges the shifting structure (16) into the feed position and the actuator (2) urges, preferably pulls, the shifting structure (16) into the reset position in which the shifting structure (16) is preferably arrested releasably;
- the shifting structure (16) is arrested releasably in a holding position against the spring force and is released by actuating the actuator (2).

5. Device according to any of the preceding claims and at least one of the following features:
- the actuator is provided accessible at the housing (1);
- the actuator is designed only to shift the mechanism (6, 7, 8);
- the actuator is a component actuation of which is required for replacing the product container (2);
- the actuator (2) is a component functional for replacing the product container (2) and being actuated automatically by replacing the product container (2).

6. Device according to any of the preceding claims, wherein the actuator (2) is represented by the product container (2) which is replaceable and effects, upon insertion into the compartment (1a), shifting of the mechanism (6, 7, 8) into the feed state.

7. Device according to the preceding claim in combination with claim 2, wherein the shifting structure (16) is arranged accessible via the compartment (1a), preferably located in the compartment (1a), and the product container (2) presses, upon insertion into the compartment (1a), against the shifting structure (16) to move the shifting structure (16) into the feed position.

8. Device according to the preceding claim, wherein
- either the shifting structure (16) forms or is disposed at or near a bottom of the compartment (1a) and the product container (2) presses with a rear face against the shifting structure (16), preferably in direct contact with the shifting structure (16), to push it into the feed position
- or the shifting structure (16) is arrested in the reset position, preferably in a positive lock, and the product container (2) releases the arrest by pressing against the shifting structure (16).

9. Device according to any of the preceding claims,
wherein the mechanism (6, 7, 8) comprises a first drive wheel (11) and a second drive wheel (12), both driven by the drive unit (5), and a first driven wheel (13) and a second driven wheel (14), both coupled with the piston rod (15),
and wherein, by shifting the mechanism (6, 7, 8), the first drive wheel (11) is drivingly connected to the first driven wheel (13) in the mechanism feed state to drive the first driven wheel (13) while the second wheels (12, 14) are disconnected, and the second drive wheel (12) is drivingly connected to the second driven wheel (14) in the mechanism reset state to drive the second driven wheel (14) while the first wheels (11, 13) are disconnected.

10. Device according to the preceding claim, wherein at least two of the wheels (11-14) can be moved axially to drivingly connect the first wheels (11, 13) and disconnect the second wheels (12, 14) in the mechanism feed state and to drivingly connect the second wheels (12, 14) and disconnect the first wheels (11, 13) in the mechanism reset state.

11. Device according to the preceding claim, further comprising a shifting structure (16) which carries the at least two of the wheels (11-14) and can be moved axially, together with the at least two of the wheels (11-14), between a feed position and a reset position,
wherein the first wheels (11, 13) are drivingly connected in the feed position and the second wheels (12, 14) are drivingly connected in the reset position of the shifting structure (16).

12. Device according to any of the three preceding claims and at least one of the following features:
- the drive wheels (11, 12) are provided on a common drive shaft (7) which can preferably be moved axially;
- the driven wheels (12, 14) are provided on a common driven shaft (8) which can preferably be moved axially.

13. Device according to any of the preceding claims and at least one of the following features:
- the drive unit (5) is reversible for moving the piston rod (15) either in the feed direction (F) or the reverse direction (-F);
- the drive unit (5) comprises a rotary output shaft;
- the drive unit (5) comprises a rotary motor;
- the drive unit (5) comprises an electrical motor.

14. Device according to any of the preceding claims, wherein a drive movement of the drive unit (5) is reversed automatically when the mechanism (6, 7, 8) is shifted, preferably by actuating the actuator (2), and even more preferred the shifting structure (17) of claim 2 controls the drive unit (5) such that the drive movement is reversed automatically when the mechanism (6, 7, 8) is shifted.

15. Device according to any of claims 9 to 13, wherein the mechanism (6, 7, 30, 8) comprises a first gear wheel train (9, 10, 11,31, 13) comprising the first wheels (11, 13) for driving the piston rod (15) in the feed state and a second gear wheel train (9, 10, 12, 14) comprising the second wheels (12, 14) for driving the piston rod (15) in the reset state, wherein, for reversing the direction of movement of the piston rod (15), the number of wheels of the first gear wheel train (9, 10, 11, 31, 13) differs from the number of wheels of the second gear wheel train (9, 10, 12, 14) by an uneven integer.
